# EUROPEAN PATENT SPECIFICATION

(11) **EP 1 771 444 B1**
(45) Date of publication and mention of the grant of the patent: **21.11.2007**
(21) Application number: 05759059.8
(22) Date of filing: 20.07.2005
(51) Int. Cl.: C07D 417/12, A61K 31/4402, A61P 3/10

(54) **SALT OF OXALIC ACID WITH 5-[4-[2-(N-METHYL-N-(2-PYRIDYL)-AMINO)ETHOXY]BENZYL]THIAZOLIDIN-2,4-DIONE AND A METHOD OF ITS PREPARATION AND ITS USE**
SALZ VON OXALSÄURE MIT 5-[4-[2-(N-METHYL-N-(2-PYRIDYL)AMINO)ETHOXY]BENZYL]THIAZOLIDIN-2,4-DION UND VERFAHREN ZU DESSEN HERSTELLUNG UND DESSEN ANWENDUNG
SEL D'ACIDE OXALIQUE AVEC 5-[4-[2-(N-METHYL-N-(2-PYRIDYL)-AMINO)ETHOXY]BENZYL]THIAZOLIDIN-2,4-DIONE, ET PROCEDE DE PREPARATION ET D'UTILISATION

(30) Priority: 27.07.2004 CZ 20040844
(43) Date of publication of application: 11.04.2007
(73) Proprietor: Zentiva, a.s., 102 37 Praha 10 (CZ)
(72) Inventor: HALAMA, Ales, 530 09 Pardubice (CZ)
(74) Representative: Jirotkova, Ivana
(86) International application number: PCT/CZ2005/000058
(87) International publication number: WO 2006/010345

(56) References cited:
- WO-A-00/63205
- WO-A-02/26737
- WO-A-94/05659
- WO-A-20/05049532

## Description

### Technical Field

The invention concerns a new salt of rosiglitazone, i.e. of 5-[4-[2-(*N*-methyl-*N*-(2-pyridyl)amino)-ethoxy]benzyl]thiazolidin-2,4-dione, with oxalic acid, including a method of its preparation. The salt can be used to prepare pharmaceuticals for treating of hyperglycemia of patients with *diabetes mellitus* of type 2.

### Background Art

Rosiglitazone, chemically 5-[4-[2-(*N*-methyl-*N*-(2-pyridyl)amino)ethoxy]benzyl]-thiazolidin-2,4-dione of formula I, is a known anti-hyperglycemic agent, which was first described in the patent EP306228 (1989) of Beecham. Rosiglitazone is in praxis used in the form of salts, especially with maleic acid (WO 94/05659 A1, formula **II**). Recently, a number of crystalline modifications of rosiglitazone maleate **II** and of its hydrates have been known (WO 2000/64893 A2 and WO 2000/064892 A2, WO 2002/026737 A1, WO 99/31095 A1, WO 99/31094 A1 and WO 99/31093 A1). A number of other addition salts is also known, both with mineral acids and with strong organic acids (WO 02/20519 A1, WO 02/20518 A1).

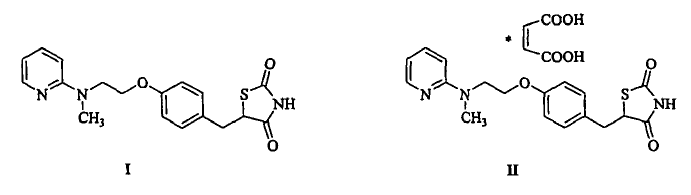

The solution being described allows preparing a hitherto undisclosed salt of rosiglitazone with oxalic acid in quality required for pharmaceutical substances. It can be assumed that the obtained salt shows anti-hyperglycemic activity, as other salts of rosiglitazone do.

### Disclosure of Invention

The invention concerns a salt of oxalic acid with 5-[4-[2-(*N*-methyl-*N*-(2-pyridyl)amino)ethoxy]-benzyl]thiazolidin-2,4-dione, of formula **III**, and a method of its preparation, which is characterized by the reaction of 5-[4-[2-(*N*-methyl-*N*-(2-pyridyl)-amino)ethoxy]benzyl]thiazolidin-2,4-dione of formula I with oxalic acid, which is carried out in an appropriate organic solvent. The employed process carried out without any problem, in which chemical purity of 99.5% and higher, with content of individual impurities bellow 0.1%, is described by the equation in Scheme 1. An economically viable method of preparation of the salt has also been found, which can be used also on the production scale.

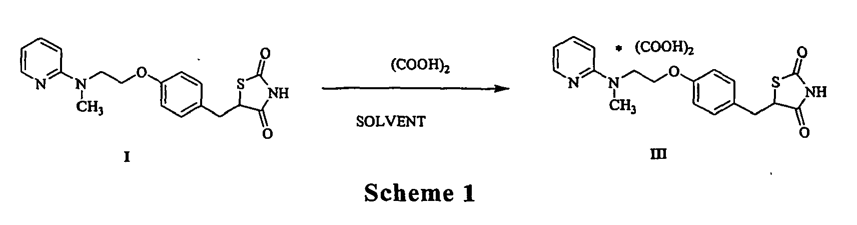

From the chemical point of view, our product is a salt that contains a component described by formula **I** and oxalic acid ((COOH)₂) in the ratio 1:1, i.e. rosiglitazone oxalate. The chemical structure of the salt we have obtained can be described by formulae **III**, **III-a**, **III-b** and **III-c,** which are equivalent to each other. However, for the sake of simplicity, formula III will be used further on in the text.

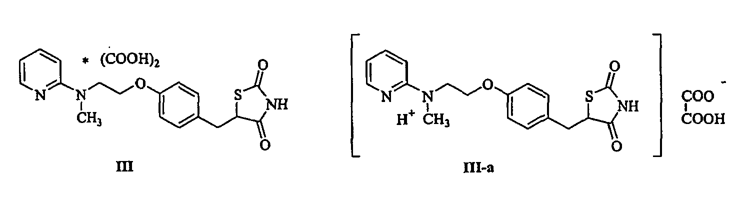

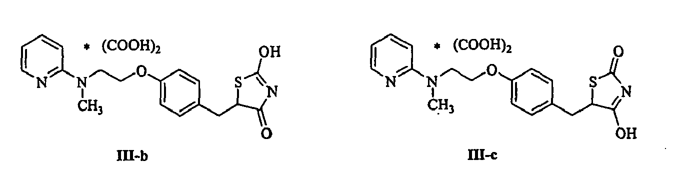

A method of preparation of the compound of formula **III,** is characterized by using of an appropriate organic solvent. The choice of the solvent depends on solubility of the starting substance and the product. Alcohols (e.g. methanol, ethanol, 1-propanol, 2-propanol, butanols), esters of carboxylic acids (e.g. ethyl acetate), ethers (e.g. dioxane, tetrahydrofuran, diethyl ether), ketones (e.g. acetone, cyclobutanone), acetonitrile, their arbitrary mixtures and mixtures with water in any ratios can be used as solvents. In the advantageous embodiment of the present invention, the solvent is ethanol or its mixture with water at any ratio. It was demonstrated, that the selected approach leads to the crystalline salt of formula **III**, which contains the compound I and oxalic acid in the ratio 1:1.

The chemical stability of the salt of formula **III** can be seen from example 3, where from the reaction of two equivalents of the free base of rosiglitazone **I** with one equivalent of oxalic acid does not arise the expected salt of formula **IV**, but an equimolar mixture of the salt of formula **III** and the free base of rosiglitazone of formula **I**.

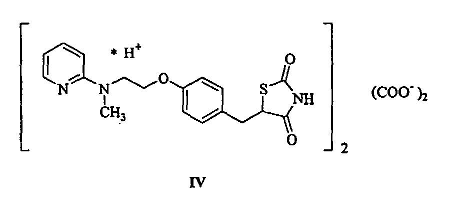

If an excess of oxalic acid is used in reaction of the compound of formula **I** with oxalic acid (Example 4), there is arisen a crystalline salt of formula **III** as the exclusive product, which contains the compound described with formula **I** and oxalic acid in the ratio 1:1. The above-described effects have an extraordinary impact on obtainable purity and stability of the product.

An advantageous form of the salt of formula **III** is the crystalline form, which is chemically stable, chemically very pure (above 99.5% according to HPLC), well soluble in water and in aqueous solutions of hydrochloric acid, which can be prepared in high yields in the defined crystalline modification, and which is characterized by suitable particle size for further processing. The crystalline form of the salt of formula **III**, prepared by us, meets these requirements.

The crystalline structure of this salt of formula **III** has been uniquely characterized by the results of the following analytical methods: X-ray powder diffraction (XRPD), melting point, differential scanning calorimetry (DSC), ¹³C CP-MAS NMR and FT-IR spectroscopy. The results of analyses are presented in Examples and enclosed Figures.

Contrary to some other salts of rosiglitazone, the above-described crystalline salt of formula **III** is well soluble in water and in aqueous solutions of hydrochloric acid. Especially the solubility in solutions of hydrochloric acid is very important considering the acidobasic conditions in the digestive system (especially in the stomach). It offers indication of the solubility of the substance after it is digested, which is a very important factor for evaluating pharmaceutical effectiveness of the substance. A comparison of the solubility of the salt of the compound of formula **III** in acidic environment with some of several other rosiglitazone salts is documented in Example 7. For example, 1 g of the crystalline salt of formula **III** can be completely dissolved in 40 ml of 0.1 M aqueous solution of hydrochloric acid at 25 °C. However, neither the same amount of rosiglitazone hydrochloride prepared according to WO 2000/063205 nor the same amount of pharmaceutically used rosiglitazone maleate of formula **II** prepared according to WO 94/05659 could be dissolved under the same conditions.

It is especially advantageous to prepare the crystalline form of the salt of the compound of formula **III** according to the invention if the solvent is ethanol or its mixture with water at any ratios. The process yields exclusively a defined crystalline modification (DSC and XRPD) and defined size of particles; in addition the process is characterized by high yields, which can be achieved reproducibly. The listed properties of the crystalline salt of formula **III** are very advantageous for its production and pharmaceutical use.

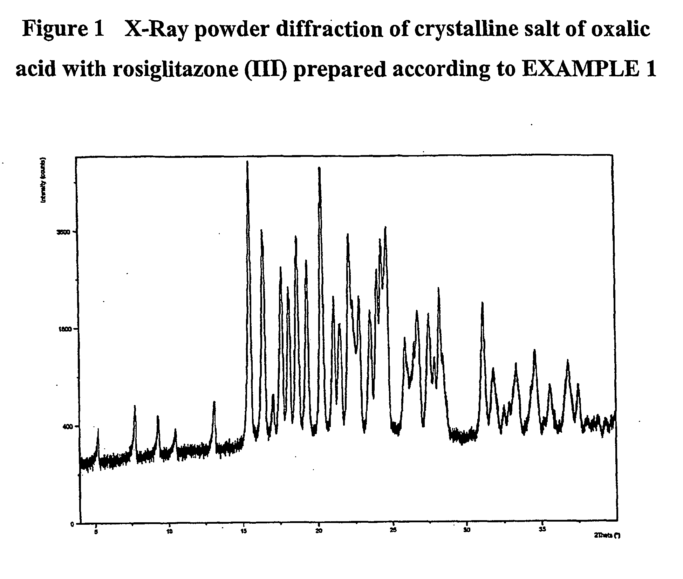

The described process produces repeatedly, with yields of 85 to 95%, a crystalline salt of oxalic acid with 5-[4-[2-(*N*-methyl-*N*-(2-pyridyl)amino)ethoxy]benzyl]-thiazolidin-2,4-dione, of formula **III**, which had in all the cases the same and uniquely defined crystalline modification, which is documented by the results of comparative XRPD measurements in Figure 1.

This procedure allows obtaining of a crystalline product with particle of 1 to 80 µm, more than 95 % of particles being characterized by their maximum dimension smaller than 50 µm. This result was obtained without using complicated grinding or other disintegration common in the pharmaceutical technology. The particle size is extraordinarily important for pharmaceutical use of the product and it is often very labor-intensive or impossible to obtain a product that would have a suitable size of particles. Methods of manufacture that produce such a product directly are, therefore, unique and exceptionally desirable.

The obtained salt of oxalic acid with 5-[4-[2-(*N*-methyl-*N*-(2-pyridyl)amino)ethoxy]-benzyl]thiazolidin-2,4-dione of formula **III,** can be used to prepare pharmaceutically applicable compositions, especially drugs with anti-hyperglycemic effect.

The subject matter of the invention is explained in more detail in the following examples, which, however, do not limit the extent of the invention defined in the claims in any respect.

### Brief Description of Drawings

Fig. 1 is the X-Ray powder diffraction of the crystalline salt of oxalic acid with rosiglitazone (III) prepared according to Example 1.
Fig. 2 depicts the DSC curve of the crystalline salt of oxalic acid with rosiglitazone (III) prepared according to Example 1.
Fig. 3 is the ¹³C CP-MAS NMR spectrum of the crystalline salt of oxalic acid with rosiglitazone (III) prepared according to Example 1.
Fig. 4 is the Fourier transform infrared spectra (FT-IR spectra) of the crystalline salt of oxalic acid with rosiglitazone (III) prepared according to Example 1.
Fig. 5 depicts a diagram of distribution of particles sizes according to the maximum dimension (MaxFeret) for the crystalline salt of oxalic acid with rosiglitazone (III) prepared according to Example 1.

### Examples

### EXAMPLE 1

20 g of the free base of rosiglitazone of formula **I** and 7.4 g of oxalic acid dihydrate was dissolved in 600 ml of ethanol. The mixture was stirred and heated to the reflux. Thus obtained solution was left to cool down freely under stirring for 5 hours. The precipitated product was filtered off and washed with 50 ml of ethanol and dried in a vacuum drier. The crystalline salt of formula **III** was obtained in 90 % yield, m.p. 155-157 °C. The chemical purity of the product was 99.76 % according to HPLC. Contents of individual impurities were always lower than 0.1 %.

### EXAMPLE 2

10 g of the free base of rosiglitazone of formula I was dissolved in 250 ml of boiling ethanol. To the boiling solution, a solution of oxalic acid dihydrate (3.7 g) in 50 ml of ethanol was added. It was left to cool down freely under stirring for 3 hours. After filtration, washing of the filtration cake with 20 ml of ethanol and drying in a vacuum drier, the crystalline salt of formula III was obtained in 90 % yield, m.p. 157-158 °C.

### EXAMPLE 3

10 g of the free base of rosiglitazone **I** was dissolved in 250 ml of boiling ethanol. To the refluxed solution, a solution of oxalic acid dihydrate (1.8 g) in 30 ml of ethanol was added. It was left to cool down freely under stirring for 3 hours. After filtration, washing of the filtration cake with 20 ml of ethanol and drying in a vacuum drier, an 11.2 g of mixture was obtained, which was the starting coumpound of formula I and the crystalline salt of formula **III**. This mixture was characterized using DS without any further assessment. Thus, it was confirmed, that the only one expected salt, the hydrogen oxalate salt of rosiglitazone, has not been formed.

### EXAMPLE 4

10 g of the free base of rosiglitazone **I** was dissolved in 250 ml of boiling ethanol. To the boiling solution, a solution of oxalic acid dihydrate (7.4 g) in 100 ml of ethanol was added. It was left to cool down freely under stirring for 3 hours. After filtration, washing of the filtration cake with 30 ml of ethanol and drying in a vacuum drier, the crystalline salt of formula III was obtained in 84 % yield, m.p. 154-157 °C.

### EXAMPLE 5

10 g of the free base of rosiglitazone of formula **I** was dissolved in 250 ml of boiling acetonitrile. To the boiling solution, a solution of oxalic acid dihydrate (3.7 g) in 50 ml of acetonitrile was added. It was left to cool down freely under stirring for 5 hours. After filtration, washing of the filtration cake with 30 ml of acetonitrile and drying in a vacuum drier, the crystalline salt **III** was obtained in 78 % yield, m.p. 153-156 °C.

### EXAMPLE 6

10 g of the free base of rosiglitazone (I) was dissolved in 250 ml of boiling isopropylalcohol. To the boiling solution, a solution of oxalic acid dihydrate (3.7 g) in 50 ml of isopropylalcohol was added. It was left to cool down freely under stirring for 5 hours. After filtration, washing of the filtration cake with 30 ml of isopropylalcohol and drying in a vacuum drier, the crystalline salt of formula **III** was obtained in 87 % yield, m.p. 155-157 °C.

### EXAMPLE 7 - solubilities of rosiglitazone salts

Solubility was determined for crystalline rosiglitazone oxalate of chemical formula **III**, which was prepared by the procedure according to Example 1. 1 g of crystalline salt of formula **III** was dissolved under stirring and at 25 °C in 20, 30, 35 a 40 ml of a 0.1M solution of hydrochloric acid. The mixture was stirred at 25 °C for five minutes. When using 20 and 30 ml of the solvent, there was obtained no solution. When using 35 ml of the solvent, there was obtained a cloudy solution. When using 40 ml of the solvent, there was obtained no cloudy solution. At the same conditions, neither dissolving of the same amount of rosiglitazone hydrochloride prepared by the procedure of WO 2000/063205 nor of the same amount of pharmaceutically used rosiglitazone maleate of formula **II** prepared according to WO 94/05659 succeeded. No solutions could be obtained even after extension of the time for dissolution and another dilution with a 0.1 M solution of hydrochloric acid.

**ANALYTICAL DATA (A-F):** The following analytical data clearly characterize the crystalline salt of rosiglitazone oxalate of chemical formula III.

### A X-Ray Powder Diffraction (XRPD)

XRPD diffraction patterns of crystalline salts of rosiglitazone oxalate **III**, which were prepared according to Example 1 are shown in **Figure 1**. The values of characteristic diffraction angles are presented in Table 1. The diffraction patterns were measured using the diffractometer Seifert 3000 XRD at the following experimental conditions:
Radiation: CoKα (λ=1.7903Å)
Monochromator: graphite
Excitation potential: 35 kV
Anodic current: 35 mA
Measured range: 4 - 40° 2θ
Step size: 0.03 2θ
Sample: flat surface with thickness of 0.5 mm

**Table 1**

| Values of characteristic diffraction angles 2θ and interplanar distances d of crystalline salt of rosiglitazone oxalate of formula **III** | | | |
|---|---|---|---|
| **(°2θ)** | **d(Å)** | **(°2θ)** | **d(Å)** |
| 15.6 | 5.68 | 23.6 | 3.77 |
| 16.5 | 5.38 | 24.0 | 3.70 |
| 17.7 | 5.00 | 24.3 | 3.66 |
| 18.2 | 4.87 | 24.7 | 3.59 |
| 18.7 | 4.73 | 25.9 | 3.44 |
| 19.4 | 4.58 | 26.8 | 3.33 |
| 20.4 | 4.35 | 26.9 | 3.31 |
| 21.2 | 4.20 | 27.5 | 3.24 |
| 22.2 | 4.00 | 28.3 | 3.15 |
| 22.9 | 3.89 | 31.2 | 2.86 |

### B Melting Point

Melting points of crystalline salts of rosiglitazone oxalate of formula **III** were measured at Kofler's block with the heating rate of the sample of 10 °C (up to 120 °C) and 4 °C (above 120 °C) per minute. The measured values of melting points range from 154 to 160 °C. Typical melting-point values are presented in Examples 1-6.

### C Differential Scanning Calorimetry (DSC)

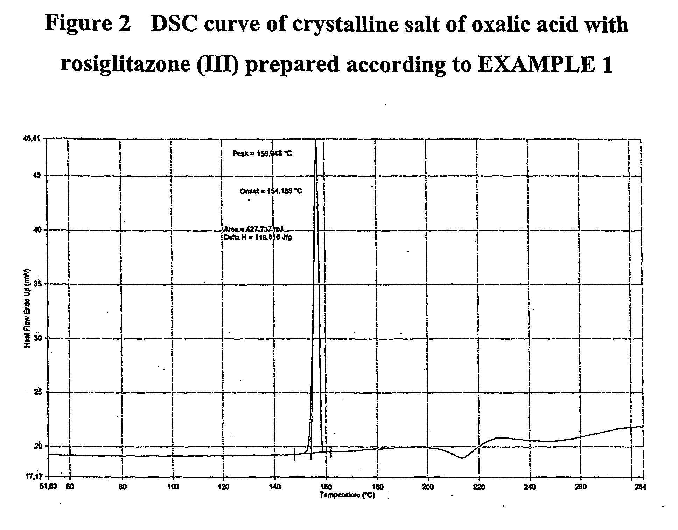

The DSC recordings were measured using the instrument Perkin Elmer PYRIS 1. The measurements were performed for samples weighting 3.6 mg. The samples were heated to temperatures 20-280 °C with the heating rate of 10 °C per minute. The measured DSC curves are presented in Figure 2. The crystalline salt of rosiglitazone oxalate of formula **III** shows a maximum at the temperature 156.5 to 157.5 °C.

### D Solid State Carbon NMR Spectra (¹³C CP-MAS NMR)

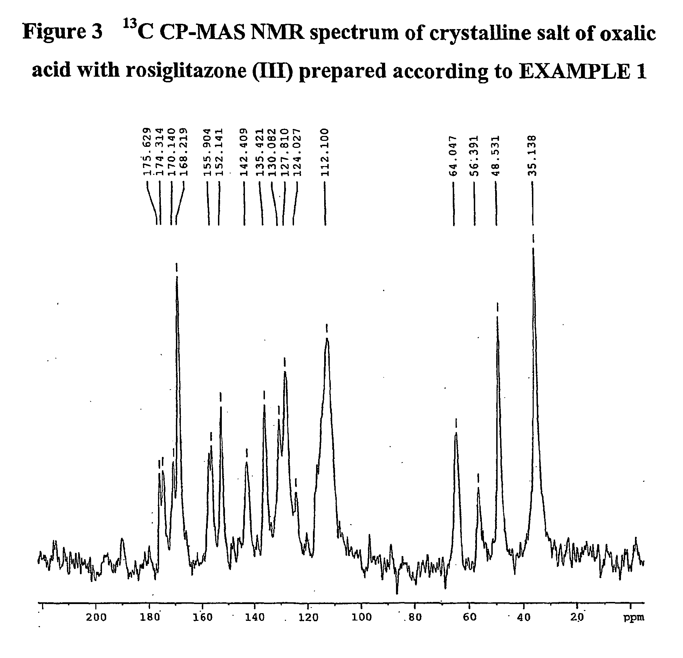

The NMR spectra of the crystalline salt of rosiglitazone oxalate III for carbon isotope ¹³C were measured using spectrometer Avance 500 Bruker with the measurement frequency 125.77 MHz using technique CP/MAS with sample rotation of 15 kHz. The obtained spectrum is presented in Figure 3. The locations of main peaks (chemical shift expressed in ppm) are: 35.1, 48.5, 56.4, 64.0, 112.1, 124.0, 127.8, 130.1, 135.4, 142.4, 152.1, 155.9, 168.2, 170.1, 174.3, 175.6.

### E Fourier Transform Infrared Spectroscopy (FT-IR)

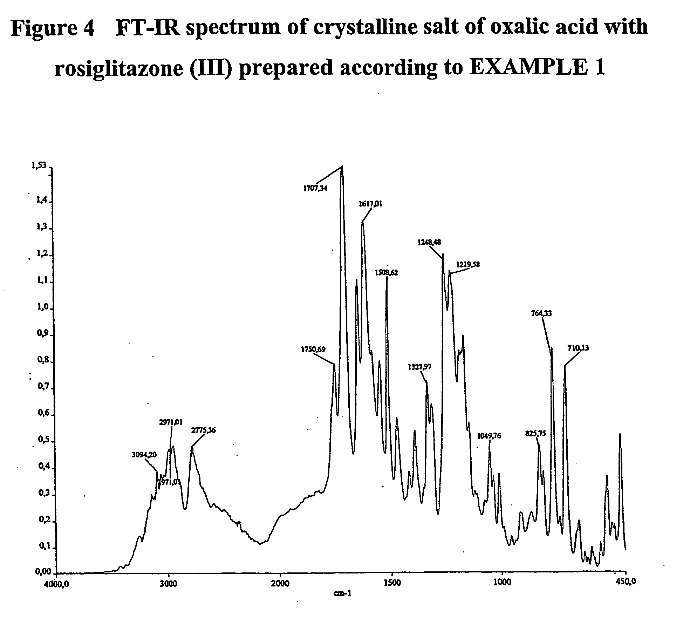

The infrared spectra of salt III were measured using the technique of KBr tablets at FT-IR spectrometer Perkin Elmer XB Spectrum with resolution of 8 cm⁻¹. The obtained spectra are presented in Figure 4. The locations of main peaks (wavenumber expressed in cm⁻¹) are: 2775, 1751, 1707, 1617, 1509, 1328, 1248, 1220, 1050, 826, 764, 710.

### F Measurement of Distribution of Particles Sizes

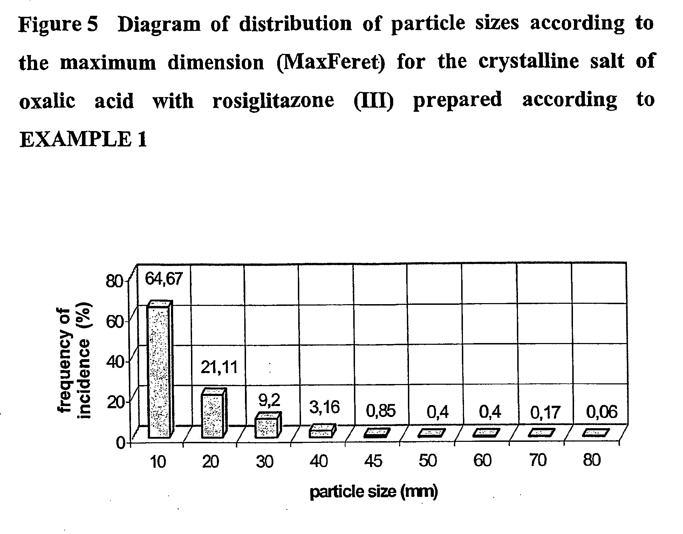

The distribution of particle sizes was measured microscopically with automatic evaluation of the measurement. A diagram of distribution of particle sizes according to the maximum dimension (MaxFeret) is presented in Figure 5. The measurement has shown that the crystalline salt of rosiglitazone oxalate **III** shows a distribution of particle sizes from 0 to 80 µm with the maximum in the interval of 0-10 µm (frequency of incidence about 65 %). More than 99 % of particles had its maximum dimension smaller than 50 µm.

## Claims

1. The salt of oxalic acid with 5-[4-[2-(*N*-methyl-*N*-(2-pyridyl)amino)ethoxy]-benzyl]thiazolidin-2,4-dione of formula **III** 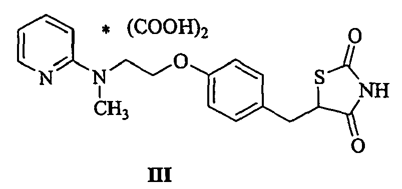 including its tautomers and solvates.

2. The salt according to claim 1, wherein the salt has a purity of 99.5 % and higher according to HPLC, with contents of individual impurities below 0.1 %.

3. The salt of oxalic acid with 5-[4-[2-(*N*-methyl-*N*-(2-pyridyl)amino)ethoxy]-benzyl]thiazolidin-2,4-dione according to claim 1 in the crystalline form.

4. The salt according to claim 3, wherein the salt is **characterized by** the following reflections in the X-ray diffraction pattern: 15.6, 16.5, 18.7, 20.4, 22.2, 24.3 (° 2θ).

5. The salt according to claim 3, wherein the salt is **characterized by** a melting point within the temperature interval of 154 to 160 °C.

6. The salt according to claim 3, wherein the salt is **characterized by** Differential Scanning Calorimetry DSC with a maximum at 156.5 to 157.5 °C.

7. The salt according to claim 3, wherein the salt is **characterized by** the following Fourier Transform Infrared Spectroscopy FT-IR (KBr) bands: 1751, 1707, 1617, 1509, 1248 (cm⁻¹).

8. The salt according to claim 3, wherein the salt is **characterized by** the following signals in ¹³C CP-MAS NMR: 35.1, 48.5, 56.4, 64.0, 112.1, 124.0, 127.8, 130.1, 135.4, 142.4, 152.1, 155.9, 168.2, 170.1, 174.3, 175.6 (ppm).

9. The salt according to claims 1 through 8, wherein the salt is soluble in water and in aqueous solutions of hydrochloric acid.

10. The salt according to claim 9, wherein the salt is **characterized in that** 1 g of the salt of formula **III** dissolves in 35 to 40 ml of 0.1M hydrochloric acid within 1 to 10 minutes.

11. A method of preparation of the salt of oxalic acid with 5-[4-[2-(N-methyl-*N*-(2-pyridyl)amino)ethoxy]benzyl]thiazolidin-2,4-dione of formula **III characterized in that** 5-[4-[2-(*N*-methyl-*N*-(2-pyridyl)amino)ethoxy]-benzyl]-thiazolidin-2,4-dione of formula **I** is reacted with oxalic acid, wherein the reaction being carried out in an organic solvent or in its mixture with water.

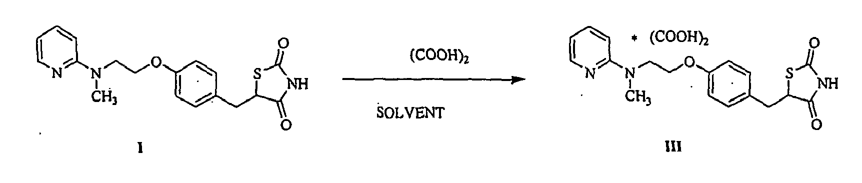

12. The method according to claim 11, **characterized in that** alcohols, esters of carboxylic acids, ethers, ketones, acetonitrile, their arbitrary mixtures and mixtures with water in any ratio are used as solvents.

13. The method according to claim 11, **characterized in that** ethanol or its mixture with water in any ratio is used as the solvent.

14. A crystalline salt obtainable according to any of claims 11 through 13, wherein said salt occurs as crystals having sizes from 1 to 80 µm, more than 95 % of the particles having their maximum dimension smaller than 50 µm.

15. A use of the salt according to claims 1 through 10 or 14 for the manufacture of pharmaceutically applicable compositions.

16. The use of the salt according to claims 1 through 10 or 15 for the manufacture of a medicament having anti-hyperglycemic effect.

## Patentansprüche

1. Salz der Oxalsäure mit 5-[4-[2-(*N*-Methyl-*N*-(2-pyridyl)amino)ethoxy]-benzyl]thiazolidin-2,4-dion der Formel **III,** 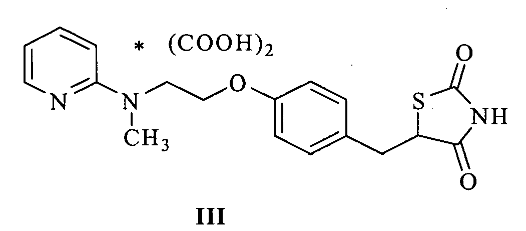 einschließlich deren Tautomere und Solvate.

2. Salz nach Anspruch 1, wobei dieses Salz gemäß HPLC die Reinheit 99,5 % und höher hat, mit Anteilen der individuellen Unreinigungen unter 0,1 %.

3. Salz der Oxalsäure mit 5-[4-[2-(*N*-Methyl-*N*-(2-pyridyl)amino)ethoxy]-benzyl]thiazolidin-2,4-dion nach Anspruch 1 in kristalliner Form.

4. Salz nach Anspruch 3, wobei dieses Salz durch die folgenden Reflexionen im Röntgen-Pulverbeugungsaufnahme charakterisiert ist: 15,6, 16,5, 18,7, 20,4, 22,2, 24,3 (° 2θ).

5. Salz nach Anspruch 3, wobei dieses Salz durch den Schmelzpunkt in einem Temperaturbereich zwischen 154 bis 160 °C charakterisiert ist.

6. Salz nach Anspruch 3, wobei dieses Salz in der Differential-Scanning-Kalorimetrie DSC durch Maximum bei 156,5 bis 157,5 °C charakterisiert ist.

7. Salz nach Anspruch 3, wobei dieses Salz durch die folgenden Banden im IR-Spektrum mit Fourier-Transformation FT-IR in KBr charakterisiert ist: 1751, 1707, 1617, 1509, 1248 (cm⁻¹).

8. Salz nach Anspruch 3, wobei dieses Salz durch die folgenden Signale in ¹³C CP-MAS NMR charakterisiert ist: 35,1, 48,5, 56,4, 64,0, 112,1, 124,0, 127,8, 130,1, 135,4, 142,4, 152,1, 155,9, 168,2, 170,1, 174,3, 175,6 (ppm).

9. Salz nach Ansprüchen 1 bis 8, wobei dieses Salz im Wasser und in wäßrigen Lösungen der Salzsäure löslich ist.

10. Salz nach Anspruch 9, wobei dieses Salz dadurch charakterisiert ist, dass 1 g des Salzes nach Formel **III** in 35 bis 40 ml der 0,1M Salzsäure innerhalb von 1 - 10 Minuten aufgelöst wird.

11. Verfahren zur Herstellung des Salzes der Oxalsäure mit 5-[4-[2-(N-Methyl-N-(2-pyridyl)amino)ethoxy]benzyl]thiazolidin-2,4-dion der Formel **III**, **dadurch gekennzeichnet, dass** 5-[4-[2-(*N*-Methyl-*N*-(2-pyridyl)amino)ethoxy]benzyl]-thiazolidin-2,4-dion der Formel I mit der Oxalsäure umgesetzt wird, wobei die Umsetzung in organischem Lösungsmittel oder ihrem Gemisch mit Wasser durchgeführt wird.

12. Verfahren nach Anspruch 11, **dadurch gekennzeichnet, dass** als Lösungsmittel Alkohole, Karboxylsäureestern, Äther, Ketone, Azetonitril, deren beliebige Mischungen und Mischungen mit Wasser in beliebigen Verhältnissen eingesetzt werden.

13. Verfahren nach Anspruch 11, **dadurch gekennzeichnet, dass** als Lösungsmittel Ethanol oder sein Gemisch mit Wasser in beliebigen Verhältnissen eingesetzt wird.

14. Kristallines Salz, herstellbar nach einem der Ansprüche 11 bis 13, wobei dieses Salz in Kristallen in Größe zwischen 1 - 80 µm vorkommt, wobei mehr als 95 % der Partikel maximale Größe unter 50 µm aufweisen.

15. Verwendung des Salzes nach einem der Ansprüche 1 - 10 oder 14 zur Herstellung der pharmazeutisch anwendbaren Zusammensetzungen.

16. Verwendung des Salzes nach einem der Ansprüche 1-10 oder 15 zur Herstellung eines Medikaments mit antihyperglykämischer Wirkung.

## Revendications

1. Sel de l'acide oxalique avec la 5-[4-[2-(*N*-méthyl-*N*-(2-pyridyl)amino)éthoxy]-benzyl]thiazolidine-2,4-dione de formule III 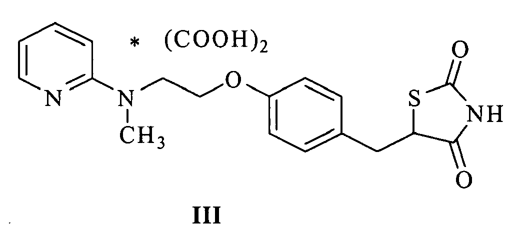 y compris ses tautomères et solvates.

2. Sel selon la revendication 1, ou le sel a la pureté 99.5 % et supérieure conformément à l'HPLC, les contenus des impuretés individuelles étant en dessous de 0,1 %.

3. Sel de l'acide oxalique avec la 5-[4-[2-(*N*-méthyl-*N*-(2-pyridyl)amino)éthoxy]-benzyl]thiazolidine-2,4-dione selon la revendication 1 sous forme cristalline.

4. Sel selon la revendication 3, ou le sel est **caractérisé par** ses réflexions dans le diffractogramme radiographique sur poudre comme suit: 15,6, 16,5, 18,7, 20,4, 22,2, 24,3 (° 2θ).

5. Sel selon la revendication 3, ou le sel est **caractérisé par** son point de fusion dans l'intervalle des températures entre 154 et 160 °C.

6. Sel selon la revendication 3, ou le sel est **caractérisé** dans la calorimétrie différentielle scannée DSC par le maximum entre 156,5 et 157,5 °C.

7. Sel selon la revendication 3, ou le sel est **caractérisé par** les bandes du spectre infrarouge avec la transformation de Fourier FT-IR (KBr): 1751, 1707, 1617, 1509, 1248 (cm⁻¹).

8. Sel selon la revendication 3, ou le sel est **caractérisé par** les signaux dans ¹³C CP-MAS NMR comme suit: 35,1, 48,5, 56,4, 64,0, 112,1, 124,0, 127,8, 130,1, 135,4, 142,4, 152,1, 155,9, 168,2, 170,1, 174,3, 175,6 (ppm).

9. Sel selon les revendications 1 à 8, ou le sel est soluble dans l'eau et dans les solutions aqueuses de l'acide chlorhydrique.

10. Sel selon la revendication 9, ou le sel est **caractérisé en ce que** 1 g du sel de formule **III** se dissoudra dans 1 à 10 minutes dans 35 à 40 ml de l'acide chlorhydrique 0,1 M.

11. Procédé de préparation du sel de l'acide oxalique avec la 5-[4-[2-(*N*-méthyl-*N*-(2-pyridyl)amino)éthoxy]benzyl]thiazolidine-2,4-dione de formule **III**, **caractérisé en ce que** l'on fait réagir la 5-[4-[2-(*N*-méthyl-*N*-(2-pyridyl)amino)éthoxy]benzyl]-thiazolidine-2,4-dione de formule **I** avec l'acide oxalique, la réaction étant réalisée dans un solvant organique ou dans son mélange avec l'eau.

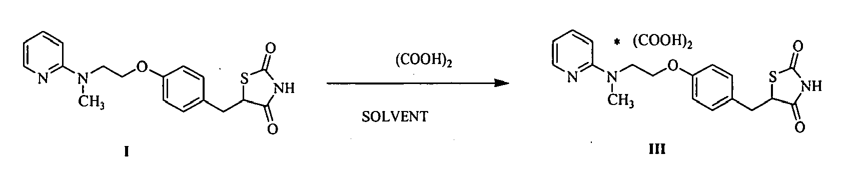

12. Procédé selon la revendication 11, **caractérisé en ce que** l'on utilise comme les solvants des alcools, des esters des acides carboxyliques, des éthers, des cétones, l'acétonitrile, leurs mélanges de toute sorte et des mélanges avec l'eau dans n'importe quels rapports.

13. Procédé selon la revendication 11, **caractérisé en ce que** l'on utilise comme le solvant l'éthanol ou bien son mélange avec l'eau dans n'importe quels rapports.

14. Sel cristallin obtenable selon une quelconque des revendications 11 à 13, ou le sel se trouve sous forme cristalline de la grosseur entre 1 et 80 µm, plus de 95 % des particules ayant la dimension maximale inférieure à 50 µm.

15. Utilisation du sel selon les revendications 1 à 10 ou 14 pour la préparation des compositions pharmaceutiquement applicables.

16. Utilisation du sel selon les revendications 1 à 10 ou 15 pour la préparation d'un médicament à l'effet antihyperglycémique.
